# EUROPEAN PATENT SPECIFICATION

(11) **EP 1 159 003 B1**
(45) Date of publication and mention of the grant of the patent: **17.11.2010**
(21) Application number: 00912065.0
(22) Date of filing: 01.03.2000
(51) Int. Cl.: A61K 39/395, A61P 11/06, C07K 16/24

(54) **ANTI-TNF ALPHA ANTIBODIES IN THERAPY OF STEROID RESISTANT ASTHMA**
ANTIKÖRPER GEGEN TNF ALPHA ZUR THERAPIE VON STEROIDRESISTENTEM ASTHMA
ANTICORPS ANTI-TNF ALPHA UTILISÉS DANS LA THÉRAPIE DE L'ASTHME RÉSISTANT AUX STÉROIDES

(30) Priority: 02.03.1999 US 260953; 17.12.1999 US 465691
(43) Date of publication of application: 05.12.2001
(73) Proprietor: CENTOCOR, INC., Malvern, PA 19355-1307 (US)
(72) Inventor: TREACY, George, Downing Town, PA 19335 (US)
(74) Representative: Kirkham, Nicholas Andrew
(86) International application number: PCT/US2000/005163
(87) International publication number: WO 2000/051637

(56) References cited:
- WO-A-91/04054
- WO-A-92/08474
- WO-A-92/16553
- WO-A-98/46642
- DE-A- 4 139 733
- R. THRALL ET AL.: "Role of tumor necrosis factor-alpha in the spontaneous development of pulmonary fibrosis in viable motheaten mutant mice." AMERICAN JOURNAL OF PATHOLOGY, vol. 151, no. 5, November 1997 (1997-11), pages 1303-1310, XP000914573 Hagerstown, MD, USA
- Y. AMRANI ET AL.: "Tumor necrosis factor alpha potentiates the increase in cytosolic free calcium induced by bradykinin in guinea-pig tracheal smooth muscle cells." COMPTES RENDUS DE L'ACADÉMIE DES SCIENCES. SÉRIE III. SCIENCES DE LA VIE., vol. 316, no. 12, December 1993 (1993-12), pages 1489-1494, XP000914554 France
- LEUNG DYM AND SZEFLER SS: "Steroid-resistant asthma" MEDICAL SCIENTIFIC UPDATE, vol. 13, no. 2, 1995, pages 1-5,
- LECKIE MARGARET J ET AL: "Effects of an interleukin-5 blocking monoclonal antibody on eosinophils, airway hyper-responsiveness, and the late asthmatic response" LANCET (NORTH AMERICAN EDITION), vol. 356, no. 9248, 2000, pages 2144-2148, ISSN: 0099-5355
- MOODY S B: "Pathogenesis and therapy management of steroid-resistant asthma" P AND T 2002 US, vol. 27, no. 3, 2002, pages 154-157, ISSN: 1052-1372
- WENZEL SALLY ET AL: "Managing severe asthma" JOURNAL OF ALLERGY AND CLINICAL IMMUNOLOGY, vol. 117, no. 3, March 2006 (2006-03), pages 508-511, ISSN: 0091-6749
- ELLIOT M.J. ET AL. ARTHRITIS & RHEUMATISM vol. 36, no. 12, December 1993, pages 1681 - 1690
- WALKER R.E. ET AL. J.INFECT.DIS. vol. 174, no. 1, July 1996, pages 63 - 68
- DOCTOR'S GUIDE, [Online] 13 May 1997 (1997-05-13), Retrieved from the Internet: URL:http://www.doguide.com/dg.nsf/PrintPri nt/815D53A771190A4285256496004B0796>
- LOCK S.H. ET AL. AM.J.RESPIR.CRIT.CARE MED. vol. 153, 1996, pages 509 - 514
- SHAH A. ET AL CLINICAL AND EXPERIMENTAL ALLERGY vol. 25, 1995, pages 1038 - 1044, XP008086096
- KIPS J.C. ET AL AM REV RESPIR DIS vol. 145, 1992, pages 332 - 336, XP008086093

## Description

### BACKGROUND OF THE INVENTION

Asthma is a chronic inflammatory disorder of the airways which usually presents in the form of recurrent episodes of wheezing, breathlessness, chest tightness and coughing, particularly at night or in the early morning. These episodes are usually associated with widespread but variable airflow obstruction that is often reversible, either spontaneously or with treatment.

Many cells and cellular elements play a role in the airway inflammation, in particular, mast cells, eosinophils, T-lymphocytes, macrophages, neutrophils and epithelial cells. The inflammation is associated with plasma exudation, oedema, smooth muscle hypertrophy, mucus plugging and epithelial changes. The inflammation also causes an associated increase in the existing bronchial hyperresponsiveness to a variety of stimuli.

Variable airflow obstruction and bronchial hyperactivity (both specific and nonspecific) are central features in symptomatic asthma. Inflammation of the airway leads to contraction of airway smooth muscle, microvascular leakage and bronchial hyperresponsiveness. When airway reactivity is high, symptoms are more severe and persistent and the magnitude of diurnal fluctuations in lung function is greater. The mechanism by which airway inflammation is related to bronchial reactivity is unclear. Recent research indicates that tumor necrosis factor alpha (TNFα), which is expressed in increased amounts in asthmatic airways, maybe associated with the increased airway hyperresponsiveness (Shah et al., Clin. Exper. Allergy, 25:1038-1044 (1995)). For example, intravenous administration of recombinant TNFα to sheep resulted in marked accentuation in histamine induced airway reactivity (Wheeler et al., J. Appl. Physiol., 68:2542-2549 (1990)) while exposure of rats to aerosolized TNFα increased airway hyperreponsiveness and induced a minor degree of airway inflammation (Kips et al., Am..Rev. Respir. Dis., 145:332-336 (1992)). In normal human subjects, inhalation of recombinant TNFα caused increased bronchial reactivity (Yates et al., Thorax, 48:1080 (1993)), while immunohistochemical analysis of bronchial biopsies from mild allergic asthmatics revealed that the increase in TNFα immunoreactivity correlated with airway hyperresponsiveness (Hosselet et al., Am. J. Respir. Crit. Care Med., 149:A957 (1994)).

Asthma is very common. It affects nearly 5% of the population in industrialized nations, yet it is underdiagnosed and undertreated. There is evidence that the incidence and prevalence of asthma are rising. These trends are occurring despite increases in the available therapies for asthma, which suggests that current methods of treating asthma are inadequate or not being utilized appropriately.

WO 92 084 74 discloses the treatment of Lung diseases by cyclosporin A or other suitable immunosuppressive agent.

WO 98 46642 discloses modified TNFα molecules, DNA encoding such modified TNFα molecules and vaccines comprising such modified TNFα molecules and DNA.

WO 91 04054 discloses a treatment of Adult Respiritory Distress Syndrome (ARDs)_{.}

### SUMMARY OF THE INVENTION

The present invention relates to the discovery that the clinical signs and symptoms associated with asthma can be ameliorated by treatment with an anti-TNFα antibody. As a result, the present invention provides uses of an anti-TNFα antibody or an antigen-binding fragment thereof for use in the treatment of steroid resistant asthma as defined in the claims.

In a preferred embodiment, the antibody is a chimeric antibody such as the cA2 monoclonal antibody.

### BRIEF DESCRIPTION OF THE DRAWINGS

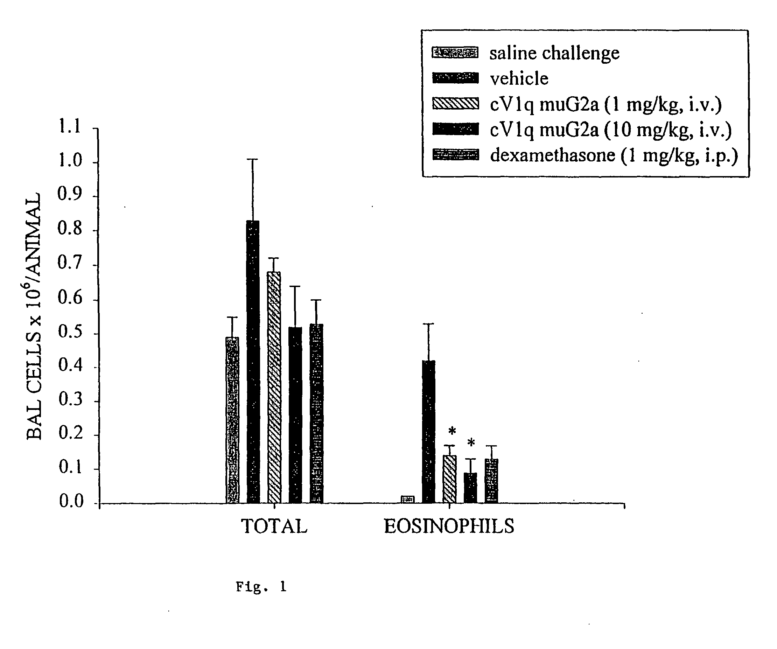

Figure 1 is a bar graph showing bronchoalveolar lavage (BAL) fluid inflammatory cell accumulation (total accumulation and eosinophil accumulation) at 72 hours following ovalbumin (OA; 5% for 20 minutes) or saline (n=10) challenge in sensitized mice treated intravenously 1 hour prior to and 24 and 48 hours following OA challenge with either (1) vehicle (PBS; n=10), (2) cV1qmuG2a antibody (1 mg/kg, n=10) or (3) cV1q muG2a antibody (10 mg/kg, n=9). An additional group of 10 mice were treated intraperitoneally 1 hour prior to and 24 and 48 hours following OA challenge with dexamethasone at 1 mg/kg. * indicates statistically significant (p<0.05) difference compared to the vehicle-treated group.

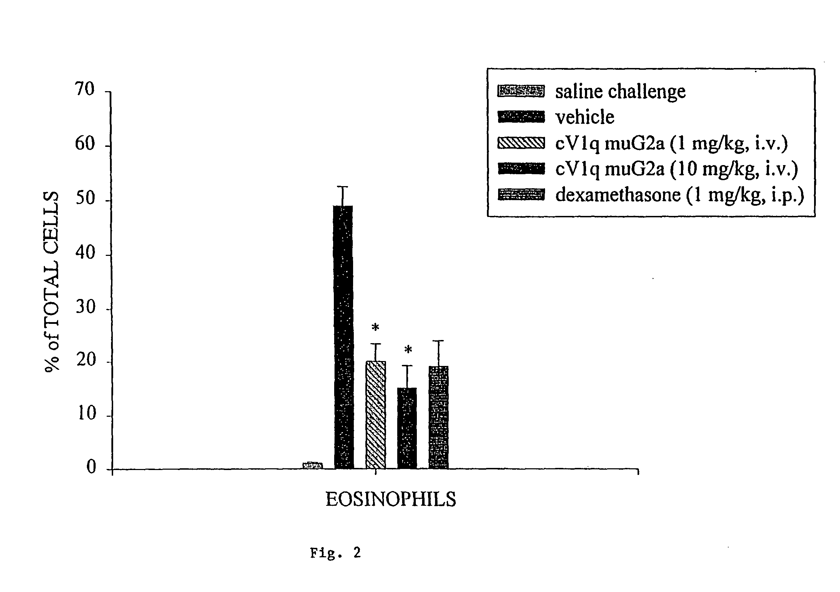

Figure 2 is a bar graph showing BAL fluid eosinophil accumulation at 72 hours following OA (5% for 20 minutes) or saline (n=10) challenge in sensitized mice treated intravenously 1 hour prior to and 24 and 48 hours following OA challenge with either (1) vehicle (PBS, n=10), (2) cV1qmuG2a antibody (1 mg/kg, n=10) or (3) cV1qmuG2a antibody (10 mg/kg, n=9). An additional group of 10 mice were treated intraperitoneally 1 hour prior to and 24 and 48 hours following OA challenge with dexamethasone at 1 mg/kg: Values are presented as a % of total cells mean ± SEM. * indicates statistically significant (p < 0.05) difference compared to the vehicle-treated group.

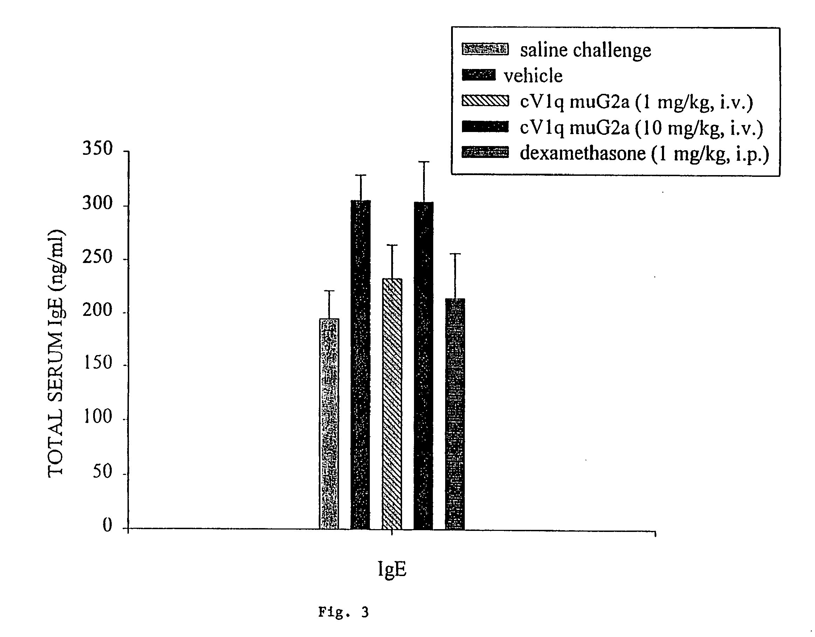

Figure 3 is a bar graph showing total serum IgE at 72 hours following OA (5% for 20 minutes) or saline (n=10) challenge in sensitized mice treated intravenously 1 hour prior to and 24 and 48 hours following OA challenge with either (1) vehicle (PBS, n=10), (2) cV1q muG2a antibody (1 mg/kg, n=10) or (3) cV1qmuG2a antibody (10 mg/kg, n=9). An additional group of 10 mice were treated intraperitoneally 1 hour prior to and 24 and 48 hours following OA challenge with dexamethasone at 1 mg/kg.

### DETAILED DESCRIPTION OF THE INVENTION

The present invention relates to the unexpected and surprising discovery that the accumulation in lungs of inflammatory cells associated with asthma, particularly bronchoalveolar lavage (BAL) eosinophils, perivascular leukocytes, interstitial leukocytes and pleural leukocytes, is significantly reduced with treatment with an anti-TNFα antibody. Airway infiltration by inflammatory cells, particularly of eosinophils into the lungs, is one of the characteristic features of asthma (Holgate, Eur. Respir. J., 6:1507-1520 (1993)). Bronchial biopsy studies performed in patients with allergic asthma show that increased numbers of eosinophils and activated T lymphocytes are present in airway tissue and BAL.

The numbers of eosinophils in peripheral blood and BAL fluid have been shown to correlate with both the degree of bronchial hyperreactivity and asthma severity (Corrigan and Kay, Immunology Today, 13:501-507 (1992)). Eosinophils store four basic proteins in their granules: major basic protein, eosinophil-derived neurotoxin, eosinophil cationic protein and eosinophil peroxidase. The release of these proteins may be responsible for airway tissue damage and bronchial hyperresponsiveness in asthmatics (Flavahan et al., Am. Rev. Respir. Dis., 138:685-688 (1988)).

T lymphocytes produce cytokines that activate cell-mediated immunity as well as humoral (IgE) immune responses. Allergic asthma is dependent on an IgE response controlled by T and B lymphocytes and activated by the interaction of antigen with mast cell-bound IgE molecules.

The results described herein demonstrate that therapy with anti-TNFα antibody is beneficial in treating asthma or airway inflammation. The results herein demonstrate that clinical signs and symptoms associated with asthma can be ameliorated by treatment with an anti-TNFα antibody. As a result, there is described methods of treating asthma or airway inflammation in an individual comprising administering an anti-TNFα antibody or an antigen-binding fragment of the anti-TNFα antibody to the individual, and methods of treating airway inflammation associated with asthma. The are described methods of reducing accumulation in lungs of inflammatory cells in an individual in need thereof, and methods of reducing accumulation in lungs of inflammatory cells associated with asthma. Symptoms, as used herein, refer to subjective feelings. For example, symptoms include when a patient complains of breathlessness, chest tightness, insomnia. Signs, as used herein, refer to what is objectively observed. For example, signs include the results of pulmonary and other laboratory tests.

### Tumor Necrosis Factor Alpha

TNFα is a soluble homotrimer of 17 kD protein subunits (Smith et al., J. Biol. Chem., 262:6951-6954 (1987)). A membrane-bound 26 kD precursor form of TNFα also exists (Kriegler et al., Cell, 53:45-53 (1988)). For reviews of TNFα, see Beutler et al., Nature, 320(6063):584-588 (1986); Old, Science, 230:630-632. (1986); and Le et al., Lab. Invest., 56:234 (1987).

TNFα is produced by a variety of cells including monocytes and macrophages, lymphocytes, particularly cells of the T cell lineage (Vassalli, Annu. Rev. Immunol., 10:411-452 (1992)), neutrophils (Dubravec et al, Proc. Natl. Acad. Sci. USA, 87:6758-6761 (1990)), epithelial cells (Ohkawara et al., Am. J. Respir. Cell. Biol., 7:985-392 (1992)) and mast cells (Shah et al., Clin. Exper. Allergy, 25:1038-1044 (1995); Gordon et al., Nature, 346:274-276 (1990); Gordon et al., J. Exp. Med, 174:103-10.7 (1991); Bradding et al., Am. J. Respir. Cell. Mol Biol., 10:471-480 (1994); Walsh et al., Proc. Natl. Acad Sci. USA, 88:4220-4224 (1991); Benyon et al., J. Immunol., 147:2253-2258 (1991); and Ohkawara et al., Am. J. Respir. Cell. Biol., 7:985-392 (1992)). Eosinophils have also been suggested as a source of TNFα (Costa et al., J. Clin. Invest., 91:2673-2684 (1993)).

### Anti-TNFα Antibodies

As used herein, an anti-tumor necrosis factor alpha antibody decreases, blocks, inhibits, abrogates or interferes with TNFα activity *in vivo.* In a preferred embodiment, the antibody specifically binds the antigen. The antibody can be polyclonal or monoclonal, and the term antibody is intended to encompass both polyclonal and monoclonal antibodies. The terms polyclonal and monoclonal refer to the degree of homogeneity of an antibody preparation, and are not intended to be limited to particular methods of production. Single chain antibodies, and chimeric, humanized or primatized (CDR-grafted antibodies, with or without framework changes), or veneered antibodies, as well as chimeric, CDR-grafted or veneered single chain antibodies, comprising portions derived from different species, and the like are also encompassed by the present invention and the term "antibody".

In a particular embodiment, the anti-TNFα antibody is a chimeric antibody. In a preferred embodiment, the anti-TNFα antibody is chimeric monoclonal antibody cA2 (or an antigen binding fragment thereof) or murine monoclonal antibody A2 (or an antigen binding fragment thereof), or has an epitopic specificity similar to that of chimeric antibody cA2, murine monoclonal antibody A2, or antigen binding fragments thereof, including antibodies or antigen binding fragments reactive with the same or a functionally equivalent epitope on human TNFα as that bound by chimeric antibody cA2 or murine monoclonal antibody A2, or antigen binding fragments thereof. Antibodies with an epitopic specificity similar to that of chimeric antibody cA2 or murine monoclonal antibody A2 include antibodies which can compete with chimeric antibody cA2 or murine monoclonal antibody A2 (or antigen binding fragments thereof) for binding to human TNFα. Such antibodies or fragments can be obtained as described above. Chimeric antibody cA2, murine monoclonal antibody A2 and methods of obtaining these antibodies are also described in Le et al., U.S. Patent No. 5,656,272; Le et al., U.S. Patent No.5,698,195; U.S. Patent No. 5,919,452; Le, J. et al., International Publication No. WO 92/16553 (published October 1, 1992); Knight, D.M. et al., Mol. Immunol., 30:1443-1453 (1993); and Siegel, S.A. et al., Cytokine, 7(1):15-25 (1995). Chimeric antibody cA2 is also known as infliximab and REMICADE^{®}.

Chimeric antibody cA2 consists of the antigen binding variable region of the high-affinity neutralizing mouse anti-human TNFα IgG1 antibody, designated A2, and the constant regions of a human IgG1, kappa immunoglobulin. The human IgG1 Fc region improves allogeneic antibody effector function, increases the circulating serum half-life and decreases the immunogenicity of the antibody. The avidity and epitope specificity of the chimeric antibody cA2 is derived from the variable region of the murine antibody A2. In a particular embodiment, a preferred source for nucleic acids encoding the variable region of the murine antibody A2 is the A2 hybridoma cell line.

Chimeric A2 (cA2) neutralizes the cytotoxic effect of both natural and recombinant human TNFα in a dose dependent manner. From binding assays of chimeric antibody cA2 and recombinant human TNFα, the affinity constant of chimeric antibody cA2 was calculated to be 1.04x10¹⁰M⁻¹. Preferred methods for determining monoclonal antibody specificity and affinity by competitive inhibition can be found in Harlow, et al., Antibodies: A Laboratory Manual, Cold Spring Harbor Laboratory Press, Cold Spring Harbor, New York, 1988; Colligan et al., eds., Current Protocols in Immunology, Greene Publishing Assoc. and Wiley Interscience, New York, (1992, 1993); Kozbor et al., Immunol. Today, 4:72-79 (1983); Ausubel et al., eds. Current Protocols in Molecular Biology, Wiley Interscience, New York (1987, 1992, 1993); and Muller, Meth. Enzymol., 92:589-601 (1983).

In a particular embodiment, chimeric antibody cA2 is produced by a cell line designated c168A and murine monoclonal antibody A2 is produced by a cell line designated c134A.

Additional examples of anti-TNFα antibodies (or antigen-binding fragments thereof) are described in the art (see, e.g., U.S. Patent No. 5,231,024; Möller, A. et al., Cytokine, 2(3):162-169 (1990); Rathjen et al., International Publication No. WO 91/02078 (published February 21, 1991); Rubin et al., EPO Patent Publication No. 0 218 868 (published April 22, 1987); Yone et al., EPO Patent Publication No. 0 288 088 (October 26, 1988); Liang, et al., Biochem. Biophys. Res. Comm., 137:847-854 (1986); Meager, et al., Hybridoma, 6:305-311 (1987); Fendly et al., Hybridoma, 6:359-369 (1987); Bringman, et al., Hybridoma, 6:489-507 (1987); and Hirai, et al., J. Immunol. Meth., 96:57-62 (1987).

Suitable antibodies are available, or can be raised against an appropriate immunogen, such as isolated and/or recombinant antigen or portion thereof (including synthetic molecules, such as synthetic peptides) or against a host cell which expresses recombinant antigen. In addition, cells expressing recombinant antigen, such as transfected cells, can be used as immunogens or in a screen for antibody which binds receptor (see e.g., Chuntharapai et al., J. Immunol., 152: 1783-1789 (1994); and Chuntharapai et al., U.S. Patent No. 5,440,021).

Preparation of immunizing antigen, and polyclonal and monoclonal antibody production can be performed using any suitable technique. A variety of methods have been described (see e.g., Kohler et al., Nature, 256: 495-497 (1975) and Eur. J. Immunol., 6: 511-519 (1976); Milstein et al., Nature, 266: 550-552 (1977*);* Koprowski et al., U.S. Patent No. 4,172,124; Harlow, E. and D. Lane, 1988, Antibodies: A Laboratory Manual, (Cold Spring Harbor Laboratory: Cold Spring Harbor, NY); and Current Protocols In Molecular Biology, Vol. 2 (Supplement 27, Summer'94), Ausubel et al., Eds., (John Wiley & Sons: New York, NY), Chapter 11, (1991)). Generally, a hybridoma can be produced by fusing a suitable immortal cell line (e.g., a myeloma cell line such as SP2/0) with antibody producing cells. The antibody producing cell, preferably those of the spleen or lymph nodes, can be obtained from animals immunized with the antigen of interest. The fused cells (hybridomas) can be isolated using selective culture conditions, and cloned by limiting dilution. Cells which produce antibodies with the desired specificity can be selected by a suitable assay (e.g., ELISA).

Other suitable methods of producing or isolating antibodies of the requisite specificity, including human antibodies, can be used, including, for example, methods by which a recombinant antibody or portion thereof are selected from a library, such as, for example, by phage display technology (see, e.g., Winters et al., Annu. Rev. Immunol., 12:433-455 (1994); Hoogenboom et al., WO 93/06213; Hoogenboom et al., U.S. Patent No. 5,565,332; WO 94/13804, published June 23, 1994; Krebber et al., U.S. Patent No. 5,514,548; and Dower et al., U.S. Patent No. 5,427,908), or which rely upon immunization of transgenic animals (e.g., mice) capable of producing a full repertoire of human antibodies (see e.g., Jakobovits et al., Proc. Natl. Acad. Sci. USA, 90: 2551-2555 (1993); Jakobovits et al., Nature, 362: 255-258 (1993); Kucherlapati *et al.,* European Patent No. EP 0 463 151 B1; Lonberg et al., U.S. Patent No. 5,569,825; Lonberg et al., U.S. Patent No. 5,545,806; and Surani et al., U.S. Patent No. 5,545,807).

The various portions of single chain antibodies, chimeric, humanized or primatized (CDR-grafted antibodies, with or without framework changes), or veneered antibodies, as well as chimeric, CDR-grafted or veneered single chain antibodies, comprising portions derived from different species, can be joined together chemically by conventional techniques, or can be prepared as a contiguous protein using genetic engineering techniques. For example, nucleic acids encoding a chimeric or humanized chain can be expressed to produce a contiguous protein. See, e.g., Cabilly et al., U.S. Patent No. 4,816,567; Cabilly *et al.,* European Patent No. 0,125,023 B1; Boss et al., U.S. Patent No. 4,816,397; Boss *et al.,* European Patent No. 0,120,694 B1; Neuberger, M.S. et al., WO 86/01533; Neuberger, M.S. *et al.,* European Patent No. 0,194,276 B1; Winter, U.S. Patent No. 5,225,539; Winter, European Patent No. 0,239,400 B1; Queen et al., U.S. Patent No. 5,585,089; Queen *et al.,* European Patent No. 0,451,216 B1; Adair et al., WO 91/09967, published 11 July 1991; Adair *et al.,* European Patent No. 0,460,167 B1; and Padlan, E.A. *et al..* European Patent No. 0,519,596 A1. See also, Newman, R. et al., BioTechnology, 10: 1455-1460 (1992), regarding primatized antibody, and Huston et al., U.S. Patent No. 5,091,513; Huston et al., U.S. Patent No. 5,132,405; Ladner et al., U.S. Patent No. 4,946,778 and Bird, R.E. et al., Science, 242: 423-426 (1988)) regarding single chain antibodies.

In addition, antigen binding fragments of antibodies, including fragments of chimeric, humanized, primatized, veneered or single chain antibodies and the like, can also be produced. For example, antigen binding fragments include, but are not limited to, fragments such as Fv, Fab, Fab' and F(ab')₂, fragments. Antigen binding fragments can be produced by enzymatic cleavage or by recombinant techniques, for example. For instance, papain or pepsin cleavage can generate Fab or F(ab')₂ fragments, respectively. Antibodies can also be produced in a variety of truncated forms using antibody genes in which one or more stop codons has been introduced upstream of the natural stop site. For example, a chimeric gene encoding a F(ab')₂ heavy chain portion can be designed to include DNA sequences encoding the CH₁ domain and hinge region of the heavy chain.

Anti-TNFα antibodies suitable for use in the present invention are characterized by high affinity binding to TNFα and low toxicity (including human anti-murine antibody (HAMA) and/or human anti-chimeric antibody (HACA) response). An antibody where the individual components, such as the variable region, constant region and framework, individually and/or collectively possess low immunogenicity is suitable for use in the present invention. Antibodies which can be used in the invention are characterized by their ability to treat patients for extended periods with good to excellent alleviation of symptoms and low toxicity. Low immunogenicity and/or high affinity, as well as other undefined properties, may contribute to the therapeutic results achieved. "Low immunogenicity" is defined herein as raising significant HACA or HAMA responses in less than about 75%, or preferably less than about 50% of the patients treated and/or raising low titers in the patient treated (less than about 300, preferably less than about 100 measured with a double antigen enzyme immunoassay) (see, e.g., Elliott et al., Lancet 344:1125-1127 (1994).

As used herein, the term "antigen binding region" refers to that portion of an antibody molecule which contains the amino acid residues that interact with an antigen and confer on the antibody its specificity and affinity for the antigen. The antigen binding region includes the "framework" amino acid residues necessary to maintain the proper conformation of the antigen-binding residues.

The term antigen refers to a molecule or a portion of a molecule capable of being bound by an antibody which is additionally capable of inducing an animal to produce antibody capable of selectively binding to an epitope of that antigen. An antigen can have one or more than one epitope.

The term epitope is meant to refer to that portion of the antigen capable of being recognized by and bound by an antibody at one or more of the antibody's antigen binding region. Epitopes usually consist of chemically active surface groupings of molecules such as amino acids or sugar side chains and have specific three dimensional structural characteristics as well as specific charge characteristics. By "inhibiting and/or neutralizing epitope" is intended an epitope, which, when bound by an antibody, results in loss of biological activity of the molecule containing the epitope, *in vivo* or *in vitro,* more preferably *in vivo,* including binding of TNFα to a TNFα receptor.

### Administration

Anti-TNFα antibodies can be administered to a patient in a variety of ways. In a preferred embodiment, anti-TNFα antibodies are administered by inhalation (e.g., in an inhalant or spray or as a nebulized mist). Other routes of administration include intranasal, oral, intravenous including infusion and/or bolus injection, intradermal, transdermal (e.g., in slow release polymers), intramuscular, intraperitoneal, subcutaneous, topical, epidural, buccal, etc. routes. Other suitable routes of administration can also be used, for example, to achieve absorption through epithelial or mucocutaneous linings. Antibodies can also be administered by gene therapy, wherein a DNA molecule encoding a particular therapeutic protein or peptide is administered to the patient, e.g., via a vector, which causes the particular protein or peptide to be expressed and secreted at therapeutic levels *in vivo.* In addition, anti-TNFα antibodies can be administered together with other components of biologically active agents, such as pharmaceutically acceptable surfactants (e.g., glycerides), excipients (e.g., lactose), carriers, diluents and vehicles. If desired, certain sweetening, flavoring and/or coloring agents can also be added.

Anti-TNFα antibodies can be administered prophylactically or therapeutically to an individual prior to, simultaneously with or sequentially with other therapeutic regimens or agents (e.g., multiple drug regimens). Anti-TNFα antibodies that are administered simultaneously with other therapeutic agents can be administered in the same or different compositions.

Anti-TNFα antibodies can be formulated as a solution, suspension, emulsion or lyophilized powder in association with a pharmaceutically acceptable parenteral vehicle. Examples of such vehicles are water, saline, Ringer's solution, dextrose solution, and 5% human serum albumin. Liposomes and nonaqueous vehicles such as fixed oils can also be used. The vehicle or lyophilized powder can contain additives that maintain isotonicity (e.g., sodium chloride, mannitol) and chemical stability (e.g., buffers and preservatives). The formulation can be sterilized by commonly used techniques. In a preferred embodiment, anti-TNFα antibodies are administered via the intranasal route (by inhalation). Suitable pharmaceutical carriers are described in Remington's Pharmaceutical Sciences.

A "therapeutically effective amount" of anti-TNFα antibody or antigen-binding fragment is defined herein as that amount, or dose, of anti-TNFα antibody or antigen-binding fragment that, when administered to an individual, is sufficient for therapeutic efficacy (e.g., an amount sufficient for significantly reducing or eliminating symptoms or signs, or both symptoms and signs, associated with asthma or airway inflammation). The dosage administered to an individual will vary depending upon a variety of factors, including the pharmacodynamic characteristics of the particular anti-TNFα antibody, and its mode and route of administration; size, age, sex, health, body weight and diet of the recipient; nature and extent of symptoms of the disease or disorder being treated, kind of concurrent treatment, frequency of treatment, and the effect desired.

The therapeutically effective amount can be administered in single or divided doses (e.g., a series of doses separated by intervals of days, weeks or months), or in a sustained release form, depending upon factors such as nature and extent of symptoms, kind of concurrent treatment and the effect desired. Other therapeutic regimens or agents can be used in conjunction the present invention. Adjustment and manipulation of established dosage ranges are well within the ability of those skilled in the art.

Once a therapeutically effective amount has been administered, a maintenance amount of anti-TNFα antibody can be administered to the individual. _ A maintenance amount is the amount of anti-TNFα antibody necessary to maintain the reduction or elimination of symptoms and/or signs achieved by the therapeutically effective dose. The maintenance amount can be administered in the form of a single dose, or a series of doses separated by intervals of days or weeks (divided doses).

Second or subsequent administrations can be administered at a dosage which is the same, less than or greater than the initial or previous dose administered to the individual. A second or subsequent administration is preferably during or immediately prior to relapse or a flare-up of the disease or symptoms of the disease. For example, the second and subsequent administrations can be given between about one day to 30 weeks from the previous administration. Two, three, four or more total administrations can be delivered to the individual, as needed.

Dosage forms (composition) suitable for internal administration generally contain from about 0.1 milligram to about 500 milligrams of active ingredient per unit. In these pharmaceutical compositions the active ingredient will ordinarily be present in an amount of about 0.5-95% by weight based on the total weight of the composition.

The present invention will now be illustrated by the following Examples, which are not intended to be limiting in any way.

### EXAMPLES

### EXAMPLE 1 Effects Of A Monoclonal Anti-TNFα Antibody In A Mouse Model For Allergic Asthma.

The mouse is a standard species used in pulmonary pharmacology studies. The murine model for allergic asthma used in the experiments described herein mimics human asthma in its phenotypic characteristics. In particular, both diseases are characterized by peribronchial inflammatory cell infiltration, particularly an influx of eosinophils into lungs. Thus, the mouse model serves as a good approximation to human disease.

### Anti-TNFα Antibody

The anti-TNFα antibody cV1q muG2a was constructed by Centocor, Inc. (Malvern, PA). Hybridoma cells secreting the rat anti-murine TNFα antibody V1q were from Peter Krammer at the German Cancer Research Center, Heidelberg, Germany (Echtenacher et al., J. Immunol. 145:3762-3766 (1990)). Genes encoding the variable regions of the heavy and light chains of the V1q antibody were cloned. The cloned heavy chain was inserted into four different gene expression vectors to encode cV1q heavy chain with either a human IgG1, human IgG3, murine IgG 1 or murine IgG2a constant region. The V1q light chain gene was inserted into other expression vectors to encode either a human kappa or a murine kappa light chain constant region.

SP2/0 myeloma cells were transfected with the different heavy and light chain gene constructs. Cell clones producing chimeric V1q (cV1q) antibody were identified by assaying cell supernatant for human or murine IgG using standard ELISA assays. High-producing clones were subcloned to obtain homogenous cell lines. The murine IgG1 and IgG2a versions are referred to as C257A and C258, respectively. cV1q antibody was purified from cell supernatant by protein A chromatography.

cV1 q antibody was characterized by measuring its affinity for soluble murine TNFα, testing its ability to protect WEHI cells from murine TNFα cytotoxicity, examining its ability to neutralize or bind murine lymphotoxin, comparing the ability of the murine IgG1 and IgG2α versions to trigger complement-mediated lysis of cells expressing recombinant transmembrane murine TNFα, and examining the ability of the human IgG1 version to protect mice from lethal doses of LPS (endotoxin). cV1q binds murine TNF (muTNF) with high affinity, neutralizes muTNF in a WEHI cell cytotoxicity assay, triggers an isotype-dependent fashion complement-mediated cytotoxicity of cells expressing transmembrance muTNF. Further, cV1q did not neutralize murine lymphotoxin cytotoxic activity. The murine IgG2a version of cV1q antibody was used in the following experimental procedure, and is referred to herein as cV1q muG2a antibody.

### Experimental Procedure

Fifty female Balb/CJ mice, weighing 15-23 grams, were sensitized at 7 weeks of age by intraperitoneal injections of 10 µg ovalbumin (OA; Sigma Chemical Co., St. Louis, MO) mixed in 1.6 mg aluminum hydroxide gel suspension (Intergen, Inc., Purchase, NY) in 0.2 ml sterile saline on days 0, 7 and 14. This suspension was prepared one hour before intraperitoneal injection into each mouse.

The fifty sensitized mice were divided into five groups (10 mice/group) and treated as follows:

| Group | N | Treatment |
|---|---|---|
| 1 | 10 | Sensitized, treated with vehicle (Dulbecco's phosphate buffered saline (PBS; Centocor, Inc., Malvern, PA)) - 10 ml/kg, intravenously (i.v.), 1 hour prior to and 24 and 48 hours post OA challenge. |
| 2 | 10 | Sensitized, treated with cV1q muG2a antibody - 1 mg/kg, i.v., 1 hour prior to and 24 and 48 hours post OA challenge. |
| 3^{a} | 10 | Sensitized, treated with cV1q muG2a antibody, 10 mg/kg, i.v., I hour prior and 24 and 48 hours post OA challenge. |
| 4 | 10 | Sensitized, treated with dexamethasone (Sigma Chemical Co., St. Louis, MO) - 1 mg/kg, intraperitoneally (i.p.), 1 hour prior to and 24 and 48 hours post OA challenge. |
| 5 | 10 | Sensitized and challenged with 0.9% saline. |

| | | |
|---|---|---|
| ^{a} One animal died following first treatment with cV1q muG2a (10 mg/kg, i.v.) | | |

Mice were challenged with OA by exposure to aerosolized OA on day 21 (5% w/v in sterile saline (Baxter, Inc., Chicago, IL)) for 20 minutes. The aerosol was generated by a PARI-Master nebulizer (PARI-Respiratory, Richmond, VA). The outlet of which was connected to a small Plexiglas® chamber (Pena-Plas, Jessup, PA) containing the animals.

On day 24, seventy-two hours following OA or saline aerosol exposure, animals were retroorbitally bled and serum was collected and frozen for total serum IgE analysis. Following bleeding, animals were anesthetized with urethane (0.2 g/kg) and bronchoalveolar lavage (BAL) was performed. Briefly, the trachea was exposed and cannulated. Lungs were lavaged with 2 x 0.5 ml sterile Hank's balanced salt solution (HBSS; Gibco, Grand Island, NY) without Ca²⁺ and Mg²⁺, containing 0.1% EDTA. Lavage fluid was recovered after 30 seconds by gentle aspiration and pooled for each animal. Samples were centrifuged at 2000 rpm for 15 minutes at 5°C. Individual pellets were reconstituted with 1 ml HBSS without Ca²⁺ and Mg²⁺, containing 0.1 % EDTA. BAL total cell and differential white cell (eosinophil) counts were determined using a Technicon H1 (Roche Diagnostics, Switzerland) and cytoslide, respectively.

The serum was separated from each sample and assayed for IgE antibodies by ELISA assay. Briefly, microtiter plates were coated with 100 µl of a monoclonal rat anti-mouse IgE antibody and incubated 1 hour (±15 min) at 37°C (±2°) and overnight at 4°C (±2°). Plates were blocked with 300 µl 1% bovine serum albumin (BSA) for 1 hour (± 15 min) at 37°C (±2°). Plates were washed 5 times. Test serum was diluted 1:3, 1:6, 1:12, and 1:24 with 1% BSA in phosphate buffered saline plus 0.05% Tween-20 (PBST). 100 µl of the diluted sera was added to duplicate wells and incubated for 1.5 hours (±15 min) at 37°C (±2°). The outside wells around the plate were not used to avoid perimeter effects. 100 µl rabbit anti-mouse IgE was added to each well and the plates incubated for 1.5 hours (±15 min) at 37°C (±2°). 100 µl biotinylated goat anti-rabbit IgG was added to each well and the plates incubated for 1.5 hours (±15 min) at 37°C (±2°). Strepavidin-conjugated horseradish peroxidase (100 µl) was added to each well and the plates incubated 15 minutes (±2 min) at 37°C (±2°). Plates were washed five times with PBST between each incubation. TMB peroxidase substrate (100 µl) was added to each well and incubated at 37°C (±2°). 100µl 1M phosphoric acid was added to each well to terminate the reaction. Absorbance was read at 450 nm using a UVMax Microplate reader from Molecular Devises Corporation (Sunnyvale, CA). A standard curve using a monoclonal mouse IgE anti-DNP (SPE-7) (Sigma Chemical Co., St. Louis, MO) was run with the assay.

Total cell, eosinophil and serum IgE levels from various treatment groups were compared using an ANOVA followed by a multiple comparison test (Zar, J.H., Biostatistical Analysis, Prentice Hall: Englewood, NJ, p. 185 (1984)).

### Total Cell, Eosinophil and Serum IgE

BAL total cell, eosinophil and total serum IgE levels from the various treatment groups are shown in Table 1.

**TABLE 1: Antigen-Induced Pulmonary Inflammatory Cell Accumulation in the Mouse Individual Animal Data**

| Group Number | Animal Number | Body Weight (g) | Total Cells (x10⁶/ml) | EOS^{a} (x10⁶/ml) | EOS^{a} (% of total) | Total Serum IgE (ng/ml) |
|---|---|---|---|---|---|---|
| 1 | 1 | 22 | 0.87 | 0.50 | 57 | 328 |
| | 2 | 21 | 0.6 | 0.23 | 39 | 218 |
| | 3 | 21 | 2.19 | 1.20 | 55 | 243 |
| | 4 | 21 | 0.97 | 0.44 | 45 | 419 |
| | 5 | 21 | 0.47 | 0.14 | 30 | 305 |
| | 6 | 21 | 0.16 | 0.09 | 58 | 242 |
| | 7 | 20 | 0.80 | 0.48 | 60 | 292 |
| | 8 | 19 | 1.30 | 0.81 | 62 | 241 |
| | 9 | 19 | 0.28 | 0.12 | 44 | 366 |
| | 10 | 20 | 0.62 | 0.23 | 37 | 410 |
| 2 | 11 | 21 | 0.68 | 0.22 | 33 | 159 |
| | 12 | 20 | 0.60 | 0.16 | 27 | 124 |
| | 13 | 22 | 0.55 | 0.05 | 9 | 134 |
| | 14 | 21 | 0.92 | 0.35 | 38 | 208 |
| | 15 | 15 | 0.79 | 0.04 | 5 | 312 |
| | 16 | 23 | 0.68 | 0.12 | 18 | 345 |
| | 17 | 22 | 0.55 | 0.14 | 25 | 116 |
| | 18 | 21 | 0.68 | 0.08 | 12 | 280 |
| | 19 | 20 | 0.68 | 0.13 | 19 | 250 |
| | 20 | 21 | 0.67 | 0.11 | 16 | 402 |

| Group Number | Animal Number | Body Weight (g) | Total Cells (x10⁶/ml) | EOS^{c} (x10⁶/ml) | EOS^{c} (% of total) | Total Serum IgE (ng/ml) |
|---|---|---|---|---|---|---|
| 3 | 21 | 20 | 0.58 | 0.12 | 20 | 325 |
| | 22 | 18 | 0.67 | 0.01 | 2 | 269 |
| | 23^{a} | 19 | - | - | - | - |
| | 24 | 21 | 0.06 | 0 | 4 | 361 |
| | 25 | 20 | 0.07 | 0.02 | 22 | 316 |
| | 26 | 21 | 0.69 | 0.01 | 1 | 374 |
| | 27 | 20 | 0.55 | 0.15 | 27 | 173 |
| | 28 | 21 | 0.47 | 0.06 | 13 | 130 |
| | 29 | 21 | 1.07 | 0.33 | 31 | 502 |
| | 30^{b} | 20 | 0.02 | - | - | 502 |
| 4 | 31 | 19 | 0.57 | 0.11 | 20 | 284 |
| | 32 | 20 | 0.24 | 0.01 | 5 | 553 |
| | 33 | 21 | 0.31 | 0.01 | 2 | 545 |
| | 34 | 22 | 0.80 | 0.32 | 40 | 106 |
| | 35 | 20 | 0.31 | 0.05 | 17 | 105 |
| | 36 | 22 | 0.53 | 0.09 | 17 | 254 |
| | 37 | 20 | 0.88 | 0.43 | 49 | 136 |
| | 38 | 20 | 0.73 | 0.16 | 22 | 191 |
| | 39 | 21 | 0.51 | 0.08 | 15 | 149 |
| | 40 | 18 | 0.45 | 0.01 | 2 | 154 |
| 5 | 41 | 19 | 0.76 | 0 | 0 | 184 |
| | 42 | 21 | 0.06 | 0 | 0 | 230 |
| | 43 | 19 | 0.33 | 0 | 0 | 157 |
| | 44 | 20 | 0.42 | 0 | 0 | 262 |
| | 45 | 20 | 0.61 | 0.01 | 1 | 275 |
| | 46 | 21 | 0.70 | 0.01 | 1 | 348 |
| | 47 | 18 | 0.50 | 0 | 0 | 176 |
| | 48 | 21 | 0.59 | 0 | 1 | 133 |
| | 49 | 20 | 0.54 | 0 | 0 | 119 |
| | 50 | 19 | 0.35 | 0.01 | 2 | 63 |

| | | | | | | |
|---|---|---|---|---|---|---|
| ^{a}EOS = eosinophils ^{a} Animal found dead one day following OA challenge ^{b} Animal not included in summary data ^{c}EOS = eosinophils | | | | | | |

As illustrated in Figure 1, a 20 minute OA (5%) exposure to sensitized mice produced an approximate 2-fold increase in BAL total cells compared to saline challanged mice. Bronchoalveolar lavage eosinophils increased from virtually 0 in saline challenged mice to 0.42 ± 0.11 x 10⁶ 72 hours following OA challenge (Figure 1). The increase in BAL total cells 72 hours following OA challenge resulted primarily from the increase in eosinophils (Figure 2). As shown in Figure 3, total serum IgE levels increased by 56% following antigen challenge in sensitized mice compared to saline challenged sensitized mice.

The positive control, dexamethasone (1 mg/kg, i.p., a steroidal antiinflammatory) administered 1 hour prior to and 24 to 48 hours following OA challenge inhibited antigen-induced increases in total cells and eosinophils by 36% and 69%, respectively, compared to the vehicle-treated group (Figure 1). Dexamethasone also produced a 30% reduction in total serum IgE levels compared to the vehicle-treated group (Figure 3).

Intravenous administration of cV1q muG2a antibody, an anti-TNFα monoclonal antibody, at 1 and 10 mg/kg 1 hour prior to and 24 and 48 hours following antigen challenge (OA challenge) produced a 18% and 37% reduction, respectively in total cells compared to the vehicle-treated group (Figure 1) (0.52±0.09 x 10⁶/ml in the 10 mg/kg anti-TNFα treated group versus 0.83±0.18 x 10⁶/ml in the vehicle-treated group, NS). In addition, cV1q muG2a antibody administration at 1 and 10 mg/kg inhibited antigen-induced (OA-induced) increases in BAL eosinophils by 67% and 79%, respectively compared to vehicle-treated animals (Figure 1) (0.09±0.04 x 10⁶/ml in the 10 mg/kg anti-TNFα treated group versus 0.42±0.11 x 10⁶/ml in the vehicle-treated group, p<0.05). These results indicate that anti-TNFα antibody modulates antigen-induced pulmonary inflammatory cell accumulation in sensitized mice.

In summary, intravenous administration of cV1q muG2a antibody at 1 and 10 mg/kg at 1 hour prior to and 24 to 48 hours following OA challenge produced a 67% and 79%, respectively, reduction in BAL eosinophils compared to vehicle-treated animals. Thus, treatment with anti-TNFα antibody resulted in a significant reduction in the number of total cells and eosinophils in BAL.

### Pharmacokinetics

cV1q antibody concentrations in the serum samples were analyzed by enzyme immunoassay (EIA). Briefly, a monoclonal anti-idiotypic antibody specific for the cV1q antibody (Lot SM970109; Centocor, Inc., Malvern, PA) was coated onto a 96 well microtiter plate. The plates were then washed and blocked with 1% bovine serum albumin (BSA)/phosphate buffered saline (PBS) solution to prevent non-specific binding. This blocking solution was removed. cV1q muG2a antibody standards and diluted test samples were added to the plate for a 2 hour incubation. The plates were washed and a biotinylated version of a different anti-cV1q monoclonal antibody was added to all wells for a 2 hour incubation. The plates were washed and incubated with a horseradish peroxidase-streptavidin conjugate during a third incubation period. A final enzymatic color development step was performed using o-phenylenediamine (Sigma Chemical Co., St. Louis, MO) as a substrate. Color development was stopped with the addition of 4N sulfuric acid and the light absorbency read using a microtiter plate spectrophotometer at 490 nm. The cV1q antibody standard concentrations and their corresponding optical density values were used to construct a standard curve by a computer generated least squares fit to a four parameter equation. Sample cV1q antibody concentrations were then determined using the standard curve and the serum dilution factor for that sample.

### Results

cV1q antibody concentrations in the serum and BAL samples from the mice treated with 1 and 10 mg/kg of cV1q antibody are shown in the upper and lower sections, respectively, of Table 2.

**TABLE 2: Serum and BAL cV1q Antibody Concentrations (µg/ml)**

| cV1q muG2a Antibody (1 mg/kg, i.v.) | | | | | | | | | | | |
|---|---|---|---|---|---|---|---|---|---|---|---|
| | | | | | | | | | | | |

| Mouse | 11 | 12 | 13 | 14 | 15 | 16 | 17 | 18 | 19 | 20 | Mean ± SD |
|---|---|---|---|---|---|---|---|---|---|---|---|
| Sera | 29.7 | 28.5 | 37.6 | 23.8 | 23.4 | 26.7 | 21.0 | 31.2 | 21.4 | 27.8 | 27.1 ± 5.06 |
| BAL | .042 | .055 | <0.04 | .069 | .118 | .062 | .055 | .071 | .119 | .076 | .067 ± .035 |
| | | | | | | | | | | | |

| cV1q muG2a Antibody (10 mg/kg, i.v.) | | | | | | | | | | | |
|---|---|---|---|---|---|---|---|---|---|---|---|
| | | | | | | | | | | | |

| Mouse | 21 | 22 | 23 | 24 | 25 | 26 | 27 | 28 | 29 | 30 | Mean SD |
|---|---|---|---|---|---|---|---|---|---|---|---|
| Sera | 317 | 282 | NS | 295 | 402 | 289 | 301 | 291 | 257 | 284 | 302 ± 40.8 |
| BAL | 1.65 | .537 | NS | .626 | .176 | .391 | .429 | .306 | .851 | <0.04 | .55 ± .48 |

| | | | | | | | | | | | |
|---|---|---|---|---|---|---|---|---|---|---|---|
| NS = No Sample | | | | | | | | | | | |

Serum and bronchiolar lavage (BAL) samples from the vehicle control group (n=10) had no detectable levels of cV1q muG2a (cV1q) antibody (<0.04 µLg/ml). Following multiple (n=3) intravenous administrations of cV1q antibody at 1 mg/kg, the serum samples from these antibody treated mice (n=10) had a mean ± standard deviation cV1q antibody concentration of 27.1 ± 5.06 µg/ml; the BAL samples from these mice had a mean cV1q antibody concentration of 0.067 ± 0.035 µg/ml. The mean serum cV1q antibody concentration (n=9) following multiple (n=3) intravenous administrations of 10 mg/kg of the antibody, was 302 ± 40.8 µg/ml; the mean cV1q antibody concentration of the BAL samples from these mice was 0.55 ± 0.48 µg/ml.

The determined concentrations of cV1q antibody from the serum and BAL mouse samples confirm a dose dependent treatment with anti-TNFα antibody and that the antibody can be detected in BAL following an intravenous administration.

### EXAMPLE 2 Antigen-Induced Pulmonary Inflammatory Cell Accumulation In The Mouse: Histopathological Evaluation.

A histopathological evaluation was performed on the lungs from sensitized female Balb/CJ mice.

### Experimental Procedure

Twenty female Balb/CJ mice were sensitized at weeks of age by intraperitoneal injections of 10 µg OA (Sigma Chemical Co., St. Louis, MO) mixed in 1.6 mg aluminum hydroxide gel suspension (Intergen, Inc., Purchase, NY) in 0.2 ml sterile saline on days 0, 7 and 14. This suspension was prepared one hour before intraperitoneal injection into each mouse.

The twenty sensitized were divided into two groups (10 mice/group). One group of mice was administered intravenously 10 mg/kg cV1q muG2a antibody (Group 2) 1 hour prior to and 24 and 48 hours following OA challenge. The other group of mice was administered intravenously 10 ml/kg Dulbecco's PBS (Centocor, Inc., Malvern, PA) (vehicle) (Group 1) 1 hour prior to and 24 and 48 hours following OA challenge. Mice were challenged with OA (antigen) by exposure to aerosolized on day 21 (5% w/v in sterile saline (Baxter, Inc., Chicago, IL) for 20 minutes. The aerosol was generated by a PARI-Master nebulizer (PARI-Respiratory, Richmond, VA). The outlet of which was connected to a small Plexiglas® chamber (Pena-Plas, Jessup, PA) containing the animals.

Seventy-two hours following antigen challenge, the mice were sacrificed and the lungs were removed and filled with 10% neutral buffer formalin (NBF; Sigma Chemical Co., St. Louis, MO). Lungs were then embedded in paraffin and stained with hematoxylin and eosin. The microscopic changes were graded on a scale of one to four (minimal, slight/mild, moderate and marked/severe) depending upon the severity of the change.

### Results

Microscopic changes which could not be graded were designated as Present (P). All of the microscopic findings are presented in Table 3.

**TABLE 3: Microscopic Changes In the Lungs Of the Mice**

| Group/Treatment | Group 1 | | | | | | | | | |
|---|---|---|---|---|---|---|---|---|---|---|
| | | | | | | | | | | |
| Animal Number | 1 | 2 | 3 | 4 | 5 | 6 | 7 | 8 | 9 | 10 |
| LUNGES* | | | | | | | | | | |
| Perivascular Leukocytes | 2 | 2 | 3 | 3 | 3 | 2 | 2 | 2 | 2 | 2 |
| Perivascular Edema | 1 | | | 1 | 1 | 2 | | | | 1 |
| Mineralized Vessel, Focal | | | | | | | | | | |
| Interstitial Leukocytes | 1 | 2 | 2 | 2 | 2 | 1 | 2 | 2 | 2 | 1 |
| Interstitial Eosinophilic | 1 | 1 | 1 | 1 | 2 | 1 | 1 | 2 | 1 | 2 |
| Deposits | | | | | | | | | | |
| Alveolar Leukocytes | 1 | 1 | | 2 | 2 | 1 | 1 | 1 | 1 | 1 |
| Alveolar Macrophages | 1 | 1 | | | 1 | | | | 1 | |
| Alveolar Hemorrhage | | | | | 2 | | | | | |
| Pleural Leukocytes | 2 | 2 | 2 | 2 | 3 | 1 | 2 | 2 | 2 | |
| Pleural Macrophages | | | | | | | | | | |
| Peribronchial Lymph Node | | | | | | | | | | |
| Eosinophilic Macrophages | | | | | | | | | | |

| | | | | | | | | | | |
|---|---|---|---|---|---|---|---|---|---|---|
| * SEVERITY CODE: 1 = MINIMAL, 2 = SLIGHT, 3 = MODERATE, 4 = SEVERE, P = PRESENT | | | | | | | | | | |

**TABLE 3: Microscopic Changes In the Lungs Of the Mice (continued)**

| Group/Treatment | Group 2 | | | | | | | | | |
|---|---|---|---|---|---|---|---|---|---|---|
| | | | | | | | | | | |
| Animal Number | 1 | 2 | 3 | 4 | 5 | 6 | 7 | 8 | 9 | 10 |
| LUNGS* | | | | | | | | | | |
| Perivascular Leukocytes | 2 | 2 | 1 | 1 | 1 | 1 | 1 | 1 | 2 | 2 |
| Perivascular Edema | 1 | | | 1 | | | | | 1 | |
| Mineralized Vessel, Focal | | | P | | | | | | | |
| Interstitial Leukocytes | 1 | 1 | | | 1 | | | | | |
| Interstitial Eosinophilic | 2 | 1 | | | 1 | 1 | 2 | 1 | | |
| Deposits | | | | | | | | | | |
| Alveolar Leukocytes | 1 | 1 | | | 1 | | | | 1 | 1 |
| Alveolar Macrophages | | | | 2 | 1 | | 1 | | 1 | 1 |
| Alveolar Hemorrhage | | | | | | | | | | 2 |
| Pleural Leukocytes | 2 | 2 | | | 1 | | | | 2 | 2 |
| Pleural Macrophages | | | | | | | | | | 4 |
| Peribronchial Lymph Node | | | | | | | | | | |
| Eosinophilic Macrophages | | | | | | 3 | | | | 4 |

| | | | | | | | | | | |
|---|---|---|---|---|---|---|---|---|---|---|
| * SEVERITY CODE: 1 = MINIMAL, 2 = SLIGHT, 3 = MODERATE, 4 = SEVERE, P = PRESENT | | | | | | | | | | |

Inflammatory cell accumulations were present and enumerated in three areas of the lungs of individual mice in both test groups. Leukocyte accumulations were evaluated in the perivascular tissues surrounding the vessels in the bronchial areas, the interstitial tissues of the alveolar areas and in the pleural/subpleural tissues. A few mice in both groups had perivascular edema around the vessels in the bronchial areas. Individual mice in both groups had eosinophilic fibrin-like deposits in the capillaries of the interstitial tissues. Group 2 mice numbered 6 and 10 had moderate and severe, respectively, accumulations of eosinophilic staining macrophages in the peribronchial lymph nodes. Group 2 mouse number 10 also had severe accumulations of eosinophilic staining macrophages in the pleural tissues and peribronchial tissues admixed with inflammatory cells.

As a group, when compared to Group 1 (vehicle-treated mice), histopathological analysis showed significant reduction in the number of perivascular leukocytes, interstitial leukocytes and pleural leukocytes in the mice in Group 2 (cV1q-treated mice). These results show that anti-TNFα antibody modulates antigen-induced pulmonary inflammatory cell accumulation in sensitized mice.

### EXAMPLE 3 Infliximab Therapy For Steroid Resistant Asthma.

A 53 year old woman (N.L.) with mild chronic obstructive pulmonary disease and severe steroid dependent asthma, developed worsening of asthma over several weeks despite intensive treatment with 40 mg of prednisone orally, inhaled steroids, inhaled ipratropium, inhaled albuterol, inhaled salmeterol, oral theophylline and zileuton. Side effects from this substantial but ineffective program included weight gain, skin thinning, and bruising.

Treatment with infliximab was instituted according to Table 4.

**TABLE 4: Infliximab Infusion (Patient N.L.)**

| Day | Infusion Number | Infusion Dose (mg) | Cumulative Dose (mg) |
|---|---|---|---|
| 0 | 1 | 200 | 200 |
| 4 | 2 | 200 | 400 |
| 16 | 3 | 400 | 800 |
| 45 | 4 | 400 | 1,200 |

The patient received four infusions totaling 1,200 mg of infliximab during the treatment period.

### Results

There was a decline in asthma symptoms, cessation of nighttime awakening, a reduction in steroid use, and less reliance on inhaled medication. This improvement began within 24 hours of infliximab therapy and is documented in Table 5, the patient's diary card.

**TABLE 5: Diary Card**

| Day | Asthma Symptoms Over Past 24 Hours* | Number of Times You Woke Up Last Night Due to Asthma | Number of Puffs of Proventil Used In the Last 24 Hours | Number of Nebulization Treatments Used In the Last 24 Hours | Steroid Use (Total Dose Daily) (mg) | Peak Flow Score (ml/min)** | |
|---|---|---|---|---|---|---|---|
| | | | | | | AM | PM |
| 2 | 4 | 1 | 6 | 4 | 20 | 200 | 160 |
| 3 | 2 | 0 | 0 | 2 | 15 | 200 | 400 |
| 4 | 2 | 0 | 0 | 2 | 15 | 205 | 400 |
| 5 | 2 | 0 | 0 | 2 | 10 | 275 | 400 |
| 6 | 2 | 0 | 2 | 2 | 10 | 255 | 400 |
| 7 | 2 | 0 | 0 | 2 | 0 | 200 | 400 |
| 8 | 2 | 0 | 0 | 1 | 10 | 205 | 400 |
| 9 | 2 | 0 | 0 | 2 | 0 | 205 | 400 |
| 10 | 2 | 0 | 0 | 2 | 10 | 200 | 400 |
| 11 | 2 | 0 | 2 | 2 | 0 | 195 | 400 |
| 12 | 2 | 0 | 0 | 2 | 10 | 195 | 400 |
| 13 | 2 | 0 | 0 | 2 | 0 | 245 | 400 |
| 14 | 2 | 0 | 0 | 1.5 | 10 | 225 | 400 |
| 15 | 2 | 0 | 0 | 2 | 0 | 245 | 400 |
| 16 | 2 | 0 | 0 | 2 | 10 | 200 | 400P/350A |
| 17 | 2 | 0 | 0 | 2 | 0 | 180P/160A | 400/310 |
| 18 | 2 | 0 | 0 | 2 | 10 | 220/200 | 400/320 |
| 19 | 2 | 0 | 0 | 2 | 0 | 370/225 | 400/320 |
| 20 | 2 | 0 | 0 | 2 | 10 | 370/270 | 400/340 |
| 21 | 2 | 0 | 0 | 2 | 0 | 305/260 | 400/330 |
| 22 | 2 | 0 | 0 | 2 | 10 | 230/200 | 400/350 |
| 23 | 2 | 0 | 0 | 2 | 0 | 205/240 | 400/330 |
| 24 | 2 | 0 | 0 | 2 | 10 | 250/210 | 400/340 |
| 25 | 2 | 0 | 0 | 2 | 0 | 220/200 | 400/350 |
| 26 | 2 | 0 | 0 | 2 | 10 | 175/200 | 400/355 |
| 27 | 2 | 0 | 0 | 2 | 0 | 200/210 | 400/350 |
| 28 | 2 | 0 | 0 | 3 | 10 | 235/210 | 400/350 |
| 29 | 2 | 0 | 0 | 2 | 0 | 225/200 | 400/340 |
| 30 | 4 | 0 | 0 | 3 | 10 | 200/ 170 | 300/280 |
| 31 | 2 | 0 | 0 | 2 | 0 | 180/180 | 400/330 |
| 32 | 2 | 0 | 0 | 2 | 10 | 225/ 190 | 400/350 |
| 33 | 1 | 0 | 0 | 2 | 0 | 275/250 | 400/340 |
| 34 | 1 | 0 | 0 | 2 | 10 | 210/240 | 400/345 |
| 35 | 2 | 0 | 0 | 2 | 0 | 300/200 | 400/340 |
| 36 | 2 | 0 | 0 | 2 | 10 | 230/220 | 400/350 |
| 37 | 1 | 0 | 0 | 2 | 0 | 275/250 | 400/350 |
| 38 | 1 | 0 | 0 | 2 | 10 | 210/190 | 400/340 |
| 39 | 1 | 0 | 0 | 2 | 0 | 245/ 180 | 400/335 |
| 40 | 1 | 0 | 0 | 2 | 10 | 195/ 180 | 400/340 |
| 41 | 1 | 0 | 0 | 2 | 0 | 180/ 170 | 400/350 |
| 42 | 3 | 0 | 0 | 2 | 10 | 230/210 | 400/340 |
| 43 | 3 | 0 | 0 | 2 | 0 | 235/210 | 400/340 |
| 44 | 2 | 0 | 0 | 2 | 10 | 190/ 170 | 380/290 |
| 45 | - | 0 | - | - | 0 | 175/ 180 | - |

| | | | | | | | |
|---|---|---|---|---|---|---|---|
| * Asthma Symptom Scores were done each morning using the following scale: 0 = No symptoms during the day 1 = Symptoms for one short period during the day 2 = Symptoms for two or more short periods during the day 3 = Symptoms for most of the day which did not affect your normal daily activities 4 = Symptoms for most of the day which did affect your normal daily activities 5 = Symptoms so severe that you missed work or could not perform normal daily activities ** Peak flow scores were measured using a pediatric flow meter or both a pediatric flow meter (P) and an adult flow meter (A), as indicated. | | | | | | | |

Peak flow score is the highest velocity of air flow recorded for the patient as measured in a breathing test. In contrast to pre-treatment peak flow scores of 160 to 200 ml/min, peaks of 340 to 400 ml/min were recorded during the infliximab treatment schedule. Higher peak flow scores are better than lower scores.

During infliximab treatment, inhaled albuterol was not required. In addition, steroid use was reduced to 10 mg every other day.

The patient's quality of life was improved greatly when she received infliximab. For example, comparing the patient's quality of life responses, the patient's asthma became well controlled, and awakening at night had disappeared after the second day of infliximab treatment.

Table 6 shows the objective improvement in pulmonary function studies.

**TABLE 6: Pulmonary Function Tests (Patient N.L.)**

| Day | Forced Voluntary Capacity (FVC) | Forced Expiratory Volume in 1 second (FEV₁) | FEV₁/FVC | Forced Expiratory Flow (FEF 25-75) |
|---|---|---|---|---|
| 2 | | | | |
| (Baseline) | 54% | 33% | 56% | 13% |
| 16 | | | | |
| (Treatment) | 82% | 60% | 73% | 25% |
| 22 | | | | |
| (Treatment) | 71% | 50% | 57% | 22% |
| 28 | | | | |
| (Treatment) | 79% | 55% | 57% | 23% |
| 36 | | | | |
| (Treatment) | 87% | 66% | 61% | 32% |
| 45 | | | | |
| (Treatment) | 80% | 54% | 67% | 22% |

Forced voluntary capacity (FVC) is a measure of expiratory flow. Forced expiratory volume in 1 second (FEV₁) is the maximum amount of air that can be blown out by the patient in 1 second. Forced expiratory flow (FEF 25-75) is a velocity measurement between the first and third quarter of 1 second. Higher values are better than lower values. The FEV, values observed were the highest documented for the patient during her care in about two years.

This 53 year old female patient had prompt and sustained improvement in both signs and symptoms of treatment resistant asthma during infliximab therapy. Infliximab therapy reduced or eliminated the need for poorly tolerated or ineffective therapies.

## Claims

1. An anti-TNFα antibody or antigen-binding fragment thereof for use in the treatment of steroid resistant asthma in an individual in need thereof, said antibody comprising a human constant region, wherein said anti-TNFα antibody or antigen binding fragment competitively inhibits binding of cA2 (REMICADE^{®} infliximab) to human TNFα.

2. The antibody or antigen-binding fragment according to Claim 1, wherein said steroid resistant asthma is associated accumulation of inflammatory cells in the lungs of said individual in need.

3. The antibody or antigen-binding fragment according to Claims 1 or 2, wherein the antibody is a chimeric antibody.

4. The antibody or antigen-binding fragment according to Claim 3, wherein the chimeric antibody is the cA2 (REMICADE^{®} infliximab) monoclonal antibody.

5. The antibody or antigen-binding fragment according to Claim 1, wherein said fragment is selected from the group consisting of Fab, Fab', F(ab')₂ and Fv.

6. The antibody or antigen-binding fragment according to Claim 1, wherein said antibody or antigen-binding fragment is of immunoglobulin class IgG1.

7. The antibody or antigen-binding fragment according to Claim 1, wherein said anti-TNFα antibody or antigen-binding fragment is to be administered to the human by means of parental administration.

8. The antibody or antigen-binding fragment according to Claim 1, wherein said anti-TNFα antibody or antigen-binding fragment is to be administered to the human by means of inhalation, intranasal administration, infusion, intravenous administration, subcutaneous administration or intramuscular administration.

9. The antibody or antigen-binding fragment according to Claim 1, wherein said antibody or antigen-binding fragment is to be administered in a therapeutically effective amount, the therapeutically effective amount comprising a single or divided dose containing about 0.1 mg to 500 mg of said anti-TNFα antibody or antigen-binding fragment.

10. The antibody or antigen-binding fragment according to Claim 1, wherein said antibody or antigen-binding fragment is to be administered in a therapeutically effective amount, the therapeutically effective amount comprising four infusions totaling 1200 mg of said anti-TNFα antibody or antigen-binding fragment.

11. The antibody or antigen-binding fragment according to Claim 1, wherein said antibody comprising a human constant region and said anti-TNFα antibody or antigen-binding fragment (i) comprises the antigen-binding regions of cA2 (REMICADE^{®} infliximab) and (ii) binds to a neutralizing epitope of human TNFα with an affinity of 1.04 x 10¹⁰ liter/mole, measured as an association constant (Ka).

12. A composition comprising the antibody or antigen-binding fragment according to Claim 1 and a pharmaceutically acceptable carrier.

## Patentansprüche

1. Antikörper gegen TNFα oder dessen antigenbindendes Fragment zur Verwendung bei der Behandlung von steroidresistentem Asthma in einem Individuum mit dringendem Bedarf, wobei der Antikörper eine menschliche konstante Region umfasst und wobei der Antikörper gegen TNFα oder das antigenbindende Fragment kompetitiv die Bindung von cA2 (REMICADE ® infliximab) an menschlichen TNFα verhindert.

2. Antikörper oder antigenbindendes Fragment nach Anspruch 1, wobei das steroidresistente Asthma eine assoziierte Akkumulation entzündlicher Zellen in der Lunge des Individuums mit dringendem Bedarf ist.

3. Antikörper oder antigenbindendes Fragment nach Anspruch 1 oder 2, wobei der Antikörper ein Chimärenantikörper ist.

4. Antikörper oder antigenbindendes Fragment nach Anspruch 3, wobei der Chimärenantikörper der monoklonale Antikörper cA2 (REMICADE ® infliximab) ist.

5. Antikörper oder antigenbindendes Fragment nach Anspruch 1, wobei das Fragment aus der Gruppe ausgewählt ist, die aus Fab, Fab', F(ab')₂ und Fv besteht.

6. Antikörper oder antigenbindendes Fragment nach Anspruch 1, wobei der Antikörper oder das antigenbindende Fragment von der Immunglobolinklasse IgG1 ist.

7. Antikörper oder antigenbindendes Fragment nach Anspruch 1, wobei der Antikörper gegen TNFα oder das antigenbindende Fragment der Person mittels parenteraler Verabreichung zu verabreichen ist.

8. Antikörper oder antigenbindendes Fragment nach Anspruch 1, wobei der Antikörper gegen TNFα oder das antigenbindende Fragment der Person durch Inhalieren, intranasale Verabreichung, Infusion, intravenöse Verabreichung, subkutane Verabreichung oder intramuskuläre Verabreichung zu verabreichen ist.

9. Antikörper oder antigenbindendes Fragment nach Anspruch 1, wobei der Antikörper oder das antigenbindende Fragment in einer therapeutisch wirksamen Menge zu verabreichen ist und die therapeutisch wirksame Menge eine einzelne oder unterteilte Dosis umfasst, die etwa 0,1 mg bis 500 mg des Antikörpers gegen TNFα oder des antigenbindenden Fragments enthält.

10. Antikörper oder antigenbindendes Fragment nach Anspruch 1, wobei der Antikörper oder das antigenbindende Fragment in einer therapeutisch wirksamen Menge zu verabreichen ist und die therapeutisch wirksame Menge vier Infusionen mit insgesamt 1200 mg des Antikörpers gegen TNFα oder des antigenbindenden Fragments umfasst.

11. Antikörper oder antigenbindendes Fragment nach Anspruch 1, wobei der Antikörper eine menschliche konstante Region umfasst und der Antikörper gegen TNFα oder das antigenbindende Fragment (i) die antigenbindenden Regionen von cA2 (REMICADE® inflicimab) umfasst und (ii) sich mit einem neutralisierenden Epitop von menschlichem TNFα mit einer Affinität von 1,04 x 10¹⁰ Liter/Mol, gemessen als eine Assoziationskonstante (Ka), verbindet.

12. Komposition, die den Antikörper oder das antigenbindende Fragment nach Anspruch 1 und einen pharmazeutisch akzeptablen Träger umfasst.

## Revendications

1. Anticorps anti-TNFα ou fragment de celui-ci capable de se lier à l'antigène, destiné à être utilisé dans le traitement de l'asthme résistant aux stéroïdes chez une personne ayant besoin d'un tel traitement, ledit anticorps comprenant une région constante humaine, dans lequel ledit anticorps anti-TNFα ou fragment capable de se lier à l'antigène inhibe par compétition la liaison entre le cA2 (REMICADE® infliximab) et le TNFα humain.

2. Anticorps ou fragment capable de se lier à l'antigène selon la revendication 1, dans lequel ledit asthme résistant aux stéroïdes est associé à une accumulation de cellules inflammatoires dans les poumons de ladite personne ayant besoin d'un traitement.

3. Anticorps ou fragment capable de se lier à l'antigène selon la revendication 1 ou la revendication 2, dans lequel l'anticorps est un anticorps chimérique.

4. Anticorps ou fragment capable de se lier à l'antigène selon la revendication 3, dans lequel l'anticorps chimérique est l'anticorps monoclonal cA2 (REMICADE® infliximab).

5. Anticorps ou fragment capable de se lier à l'antigène selon la revendication 1, dans lequel ledit fragment est choisi dans le groupe formé par Fab, Fab', F(ab')₂ et Fv.

6. Anticorps ou fragment capable de se lier à l'antigène selon la revendication 1, dans lequel ledit anticorps ou fragment capable de se lier à l'antigène est de classe immunoglobulinique IgG1

7. Anticorps ou fragment capable de se lier à l'antigène selon la revendication 1, dans lequel ledit anticorps anti-TNFα ou fragment capable de se lier à l'antigène est à administrer à l'homme par voie parentérale.

8. Anticorps ou fragment capable de se lier à l'antigène selon la revendication 1, dans lequel ledit anticorps anti-TNFα ou fragment capable de se lier à l'antigène est à administrer à l'homme par inhalation, voie intranasale, infusion, voie intraveineuse, sous-cutanée ou intramusculaire.

9. Anticorps ou fragment capable de se lier à l'antigène selon la revendication 1, dans lequel ledit anticorps ou fragment capable de se lier à l'antigène est à administrer en quantité thérapeutiquement efficace, la quantité thérapeutiquement efficace consistant en une dose unique ou divisée contenant environ 0,1 mg à 500 mg dudit anticorps anti-TNFα ou fragment capable de se lier à l'antigène.

10. Anticorps ou fragment capable de se lier à l'antigène selon la revendication 1, dans lequel ledit anticorps ou fragment capable de se lier à l'antigène est à administrer en quantité thérapeutiquement efficace, la quantité thérapeutiquement efficace consistant en quatre infusions d'un total de 1200 mg dudit anticorps anti-TNFα ou fragment capable de se lier à l'antigène.

11. Anticorps ou fragment capable de se lier à l'antigène selon la revendication 1, dans lequel ledit anticorps comprenant une région constante humaine et ledit anticorps anti-TNFα ou fragment capable de se lier à l'antigène (i) comprend les régions de cA2 (REMICADE® infliximab) capables de se lier à l'antigène et (ii) se lie à un épitope de neutralisation du TNFα humain avec une affinité de 1,04 x 10¹⁰ litre/mole, mesurée comme une constante d'association (Ka).

12. Composition comprenant l'anticorps ou le fragment capable de se lier à l'antigène selon la revendication 1 et un excipient pharmaceutiquement acceptable.
